# EUROPEAN PATENT SPECIFICATION

(11) **EP 2 057 957 B1**
(45) Date of publication and mention of the grant of the patent: **03.09.2014**
(21) Application number: 08168960.6
(22) Date of filing: 12.11.2008
(51) Int. Cl.: A61B 19/02

(54) **A sterilizable pouch**
Sterilisierbarer Beutel
Poche stérilisable

(30) Priority: 12.11.2007 IE 20070824
(43) Date of publication of application: 13.05.2009
(73) Proprietor: Steripack Medical Limited, Clara Offaly (IE)
(72) Inventor: Leonard, Gary, Celbridge, County Kildare (IE); Moore, Barry, Mullingar, County Westmeath (IE); O'Brien, Aidan, Longford, County Longford (IE); Moore, Garry, Mullingar, County Westmeath (IE); Martin, Eamon, Athlone, County Westmeath (IE); Conlon, Paul, Tullamore, County Offaly (IE); Bourke, Andrew, Tullamore, County Offaly (IE)
(74) Representative: Schütte, Gearoid

(56) References cited:
- US-A- 3 761 013
- US-A- 4 367 816
- US-A- 4 550 831
- US-A- 4 874 090
- US-A- 5 230 430
- US-A- 5 551 781

## Description

### Introduction

This invention relates to methods of manufacture of sterilizable containers such as medical pouches.

A number of sterilizable plastics pouches are known in the art. For example, US 5,551,781 discloses a sterilizable pouch comprising front and rear plastics panels sealed together about their edges. An access opening in the front panel is covered by a peelable two-layered strip including a porous outer membrane and a plastic interlayer strip with an opening matching the access opening. The interlayer is tack sealed to the front panel during manufacture to stop it deforming during manufacture of the pouch. Another plastics pouch is disclosed in US 3,761,013. In this case a double-walled pouch is formed by inner and outer plies folded over and joined together and to a longitudinal closure membrane to form a composite tube stock from which pouches of a required length are cut and the free open ends sealed together by transverse sealing and cutting operations. Another sterilizable bag is disclosed in US 5,230,432 comprising a large plastics sheet folded over and closed by a filter strip to form a bag.

The invention is particularly concerned with the manufacturing of medical pouches of the type comprising two sheets of plastics material, for example low density polyethylene, which overlap and are heat sealed around their sides. Usually a bottom edge of the pouch is left open initially to allow filling of the pouch after which said bottom edge is also sealed. A peel-away cover strip is mounted over an access opening in the pouch. This cover strip is gas permeable to allow gas sterilisation of the contents of the pouch and forms a bacteriological barrier to maintain the sterility of the pouch contents after sterilisation. Cover strips used for such pouches typically comprise a surgical grade paper, or spunbonded olefin such as Tyvek (Registered Trade Mark) manufactured by The Du Pont Company. To enable such cover strips to be peelably attached to the plastics pouch, the face of the cover strip attached to the plastics pouch must be coated. For example, a heat seal coating such as polyvinylacetate is applied to a face of a Tyvek sheet to allow it to be heat sealed to polyethylene sheets forming the pouch. Such coated Tyvek material is, however relatively expensive. Further, the coating adversely affects the porosity of the cover strip, thus lengthening sterilisation times which is undesirable. To address this problem it has been proposed, see for example US 4,367,816, to mount an intermediate layer of perforated plastics material between the paper/Tyvek layer and the plastics pouch which will seal the outer paper/Tyvek layer and peelably seal with the plastics pouch material. US4367816 discloses a method for manufacturing a sterilizable plastics pouch according to the preamble of claim 1. However, probably because of a familiarity with the widely used coated Tyvek cover strips, when opening the pouch persons have a tendency to try to peel away the outer paper/Tyvek layer from the intermediate layer which makes opening the pouch extremely difficult and the outer paper/Tyvek layer tends to tear releasing fibres into the atmosphere which is undesirable in a sterile environment such as an operating theatre. A further disadvantage is the increased heat energy required for sealing all the layers of material together when the intermediate sheet is used. It is also more difficult to form a good seal between the various layers due to the extra material involved.

The present invention is directed towards overcoming these problems.

According to the invention there is provided a method for manufacturing a sterilizable plastics pouch including the steps:
forming a peel-away cover strip by heat sealing an outer gas permeable membrane to an inner gas permeable thermoplastics film layer;
overlapping the cover strip with a front thermoplastics panel of the pouch with a top edge of the front panel located intermediate a top edge and a lower edge of the cover strip;
heat sealing the cover strip to the front panel;
characterized in that the method comprises the steps of
laying a rear thermoplastics panel over the front panel and cover strip; and
heat sealing the rear panel to the front panel and the cover strip along side edge seals and a top edge seal.

In another embodiment, the method includes heat sealing the gas permeable membrane to the inner thermoplastics film layer about a periphery of the membrane and the inner thermoplastics film layer.

In a further embodiment the method includes heat sealing the cover strip to the front panel intermediate the ends of the overlapped portions spaced-apart from a free leading edge of the cover strip.

In one embodiment of the invention the method includes heat sealing the gas permeable membrane to the inner thermoplastics layer with the inner thermoplastics layer projecting outwardly of the gas permeable membrane forming a gripping flap.

In another embodiment of the invention the method includes heat sealing the gas permeable membrane to the inner thermoplastics layer along side seals and a bottom seal prior to attaching the cover strip to the rear panel.

In a further embodiment of the invention the method includes heat sealing the cover strip to the front panel by means of an inner seal which is located intermediate a top edge of the front panel and a lower leading edge of the cover strip.

In one aspect of the invention the inner seal is substantially parallel to the top edge and lower leading edge.

In a further aspect of the invention the inner thermoplastics layer has a number of scores or perforations to allow through-passage of gas.

In a still further aspect of the invention the gas permeable membrane is a surgical grade paper, the inner thermoplastics layer is a perforated peelable polyolefin film and the front and rear panels are medical grade low density polyethylene material.

In another aspect the invention provides a sterilizable plastics pouch having an access opening, a peel-away cover strip mounted across said access opening, said peel-away cover strip comprising an outer gas permeable membrane and an inner thermoplastics film layer sealed to the gas permeable membrane and peelably sealed to the pouch, said inner thermoplastics film layer having one or more openings to allow through-passage of sterilising gas, the pouch having a rear panel and a front panel which overlies the rear panel, a filling opening being formed at a bottom end of the pouch between the rear panel and the front panel, an opening formed between a top edge of the front panel and a top edge of the rear panel forms the access opening, characterised in that the outer gas permeable membrane and the inner thermoplastics layer are sealed all along a free leading edge of the cover strip which is gripped in use for peeling away the cover strip from the pouch.

In one aspect of the invention a gripping flap is formed at a leading edge of the cover strip extending outwardly from a seal securing the cover strip to the pouch for gripping the cover strip to peel the cover strip away from the pouch.

In a further aspect of the invention the gripping flap is formed by portion of the inner layer which extends outwardly of the membrane on the cover strip.

In one aspect of the invention a peripheral edge portion of the gas permeable membrane is heat sealed to the inner thermoplastics layer.

In a further aspect of the invention side edge seals and a top edge seal secure the front panel and rear panel together and secure the cover strip to the front panel and rear panel across the access opening, an inner seal substantially parallel to the top seal securing the cover strip to the front panel below the top edge of the front panel.

### Detailed Description of the invention

The invention will be more clearly understood by the following description of some embodiments thereof, given by way of example only with reference to the accompanying drawings, in which:
Fig. 1 is a perspective view of a sterilizable plastics pouch;
Fig. 2 is an exploded perspective view of the sterilizable plastics pouch;
Figs. 3 to 5 show sequential steps in the manufacture of the sterilizable plastics pouch according to a method of the invention.

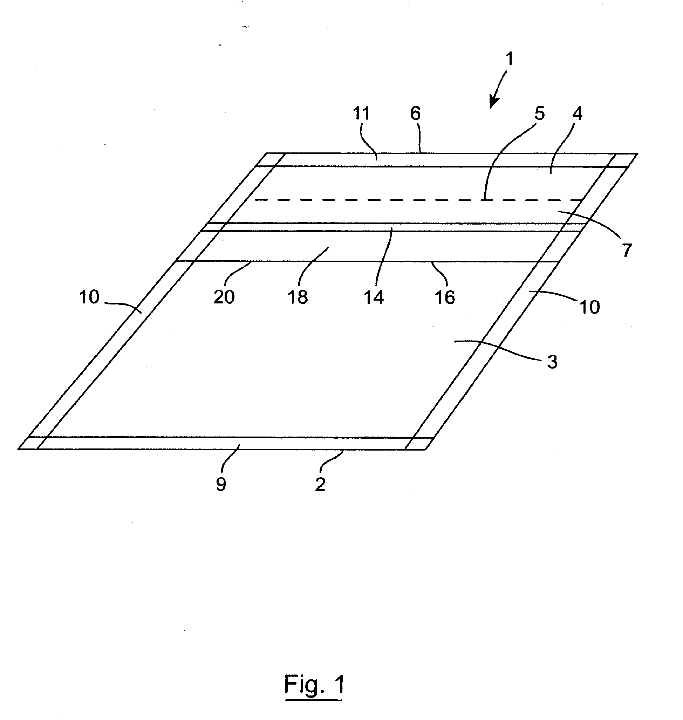

Referring to the drawings, and initially to Fig. 1 thereof, there is illustrated a sterilizable plastics pouch indicated generally by the reference numeral 1. The pouch 1 essentially comprises two sheets of a low density polyethylene material forming a rear panel 2 and a front panel 3 which overlies the rear panel 2. An opening 4 between a top edge 5 of the front panel 3 and a top edge 6 of the rear panel 2 forms an access opening. A peel-away cover strip 7 is mounted across the access opening 4. A filling opening 9 is provided at a bottom end of the pouch 1 for insertion of contents into the pouch 1 before sealing the bottom end of the pouch 1 with a heat seal.

Side edge seals 10 and a top edge seal 11 secure the front panel 3 and rear panel 2 together and secure the cover strip 7 to the front panel 3 and rear panel 2 across the access opening 4. An inner seal 14, substantially parallel to the top seal 11 secures the cover strip 7 to the front panel 3 below the top edge 5 of the front panel 3. It will be noted that the seal 14 is spaced inwardly above a lower leading edge 16 of the cover strip 7 thus providing a gripping flap 18. This flap 18 can be gripped by insertion of a person's fingers into an opening 20 between the flap 18 and a front face of the front panel 3 to readily easily grip and peel away the cover strip 7 from the front panel 3 and rear panel 2 to expose the access opening 4 for removal of the contents of the pouch 1 in use.

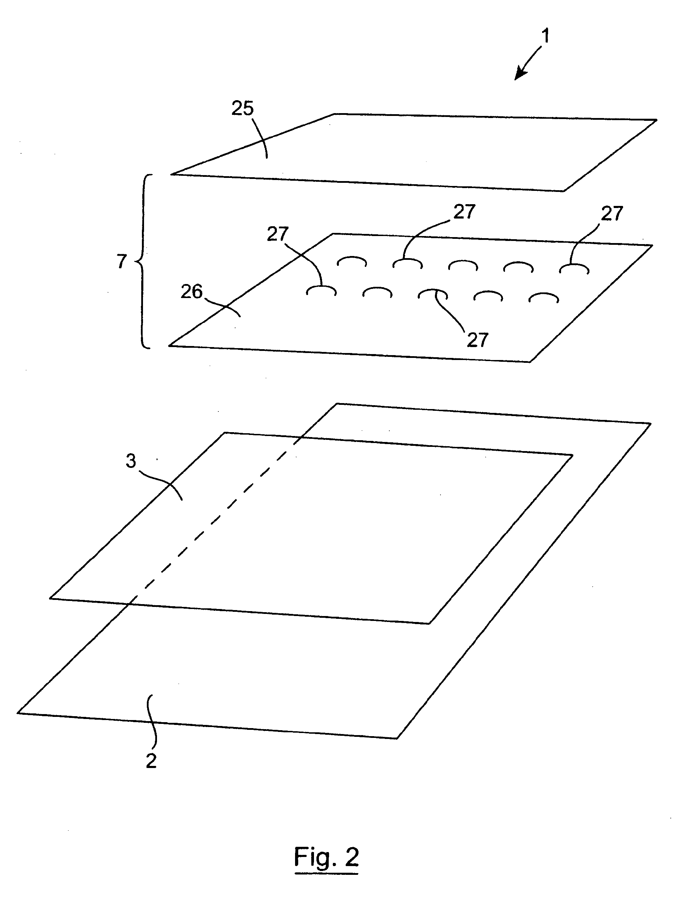

Referring in particular to Fig. 2, the peel-away cover strip 7 comprises an outer gas permeable membrane 25 which is heat sealed about its periphery to an inner thermoplastics layer 26 which has a number of scores or perforations 27 to allow through-passage of sterilising gas. The gas permeable membrane 25 may be formed from a suitable surgical grade paper or more preferably Tyvek (Registered Trade Mark). The inner thermoplastics layer 26 may conveniently be formed of a perforated peelable polyolefin film. Each of the front panel 3 and the rear panel 2 may conveniently be formed of a clear puncture resistant, medical grade low density polyethylene material.

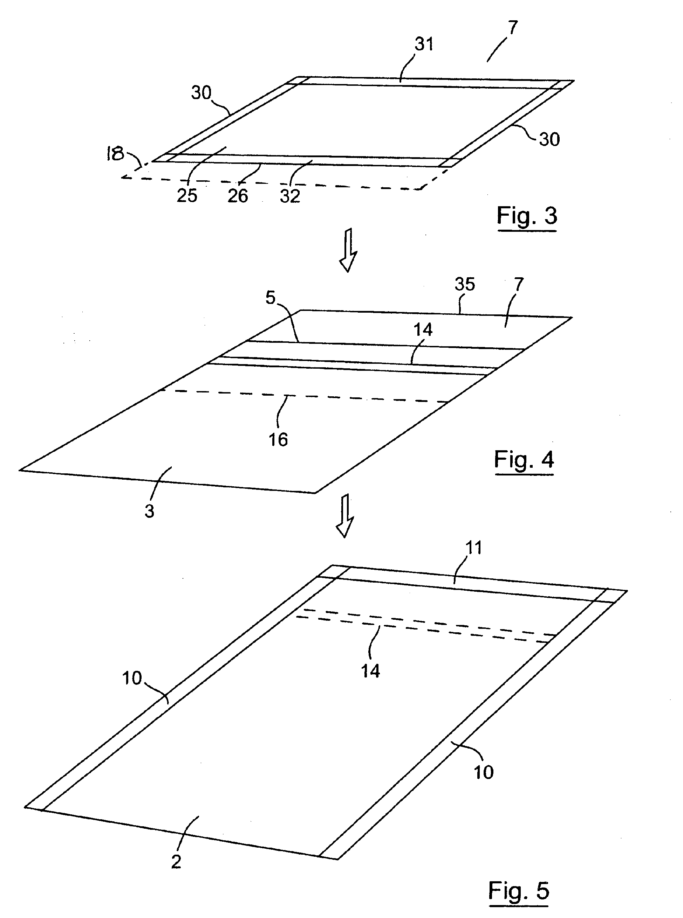

Referring now to Figs. 3 to 5, a method for manufacturing the pouch 1 according to the invention will be described. First the cover strip 7 is formed by overlying the gas permeable membrane 25 and the inner layer 26. These are sealed together about their periphery by means of side seals 30, a top seal 31 and a bottom seal 32. In some cases the top seal 31 may be omitted at this stage, this seal being effected later by the top seal 11. As the periphery of the membrane 25 is sealed to the inner layer 26 it will not be inadvertently pulled away from the inner layer 26 when opening the pouch 1. Preferably also the inner thermoplastics layer 26 extends outwardly from the membrane 25 at the bottom seal 32 to form the gripping flap 18 as shown in broken outline in Fig. 3.

The front panel 3 is overlaid on the cover strip 7 as shown in Fig. 4 with the top edge 5 of the front panel 3 locating intermediate a top edge 35 and the lower leading edge 16 of the cover strip 7. Then the cover strip 7 is heat sealed to the front panel 3 by means of the seal 14 which extends parallel to the top edge 5 of the front panel 3 and is located intermediate the top edge 5 and the lower leading edge 16 of the cover strip 7.

Then the rear panel 2 is overlaid on top of the joined front panel 3 and cover strip 7 as shown in Fig. 5 and the rear panel 2 is heat sealed to the front panel 3 and cover strip 7 by means of the side edge seals 10, 11. It will be noted that by securing the gas permeable membrane 25 and the inner layer 26 of the cover strip 7 together first and then attaching the cover strip 7 to the front panel 3 and rear panel 2 by means of the side seals 10, 11 less heat energy is required to form these side seals 10, 11 and better control of the sealing can be achieved.

The invention is not limited to the embodiments hereinbefore described which may be varied in both construction and detail within the scope of the appended claims.

## Claims

1. A method for manufacturing a sterilizable plastics pouch including the steps:
forming a peel-away cover strip (7) by heat sealing an outer gas permeable membrane (25) to a gas permeable inner thermoplastics film layer (26);
overlapping the cover strip (7) with a front thermoplastics panel (3) of the pouch with a top edge (5) of the front panel (3) locating intermediate a top edge (35) and a lower edge (16) of the cover strip (7);
heat sealing the cover strip (7) to the front panel (3);
said method being **characterized in that** it further comprises the steps of laying a rear thermoplastics panel (2) over the front panel (3) and cover strip (7); and
heat sealing the rear panel (2) to the front panel (3) and the cover strip (7) along side edge seals (10) and a top edge seal (11).

2. The method as claimed in claim 1 wherein the method includes the step of heat sealing (30, 31, 32) the gas permeable membrane (25) to the inner thermoplastics film layer (26) about a periphery of the membrane (25) and the inner thermoplastics film layer (26).

3. The method as claimed in claim 1 or claim 2 wherein the method includes the step of heat sealing (14) the cover strip (7) to the front panel (3) intermediate the ends of the overlapped portions spaced-apart from a free leading edge (16) of the cover strip (7).

4. The method as claimed in claim 1 wherein the method includes the step of heat sealing the gas permeable membrane (25) to the inner thermoplastics layer (26) with the inner thermoplastics layer projecting outwardly of the gas permeable membrane forming a gripping flap (18).

5. The method as claimed in any preceding claim wherein the method includes the step of heat sealing the gas permeable membrane (25) to the inner thermoplastics layer (26) along side seals (30) and a bottom seal (32) prior to attaching the cover strip (7) to the rear panel (2).

6. The method as claimed in any preceding claim wherein the method includes the step of heat sealing the cover strip (7) to the front panel (3) by means of an inner seal (14) which is located intermediate a top edge (5) of the front panel (3) and a lower leading edge (16) of the cover strip (7).

7. The method as claimed in claim 6 wherein the inner seal (14) is substantially parallel to the top edge (5) and lower leading edge (16).

8. The method as claimed in any preceding claim wherein the inner thermoplastics layer (26) has a number of scores or perforations (27) to allow through-passage of gas.

9. The method as claimed in any preceding claim wherein the gas permeable membrane is a surgical grade paper, the inner thermoplastics layer is a perforated peelable polyolefin film and the front and rear panels are medical grade low density polyethylene material.

## Patentansprüche

1. Verfahren zum Herstellen eines sterilisierbaren Kunststoffbeutels das folgende Schritte umfasst:
Bilden eines Abzieh-Abdeckstreifens (7) durch Heißsiegeln einer äußeren gasdurchlässigen Membran (25) an eine gasdurchlässige innere Thermoplastfilmschicht (26);
Überlappen des Abdeckstreifens (7) mit einem vorderen Thermoplastelement (3) des Beutels, wobei ein oberer Rand (5) des vorderen Elements (3) zwischen einem oberen Rand (35) und einem unteren Rand (16) des Abdeckstreifens (7) liegt;
Heißsiegeln des Abdeckstreifens (7) an das vordere Element (3);
wobei das Verfahren **dadurch gekennzeichnet ist, dass** es weiter folgende Schritte umfasst:
Legen eines hinteren Thermoplastelements (2) über das vordere Element (3) und den Abdeckstreifen (7); und
Heißsiegeln des hinteren Elements (2) an das vordere Element (3) und den Abdeckstreifen (7) entlang seitlichen Randsiegeln (10) und einem oberen Randsiegel (11).

2. Verfahren nach Anspruch 1, wobei das Verfahren den Schritt des Heißsiegelns (30, 31, 32) der gasdurchlässigen Membran (25) an die innere Thermoplastfilmschicht (26) um einen Umfang der Membran (25) und der inneren Thermoplastfilmschicht (26) umfasst.

3. Verfahren nach Anspruch 1 oder Anspruch 2, wobei das Verfahren den Schritt des Heißsiegelns (14) des Abdeckstreifens (7) an das vordere Element (3) zwischen den Enden der überlappenden Abschnitte beabstandet von einem freien vorderen Rand (16) des Abdeckstreifens (7) umfasst.

4. Verfahren nach Anspruch 1, wobei das Verfahren den Schritt des Heißsiegelns der gasdurchlässigen Membran (25) an die innere Thermoplastschicht (26) umfasst, wobei die innere Thermoplastschicht von der gasdurchlässigen Membran nach außen vorsteht und eine Greiflasche (18) bildet.

5. Verfahren nach einem der vorangehenden Ansprüche, wobei das Verfahren den Schritt des Heißsiegelns der gasdurchlässigen Membran (25) an die innere Thermoplastschicht (26) entlang seitlichen Siegeln (30) und einem unteren Siegel (32) vor dem Anbringen des Abdeckstreifens (7) an dem hinteren Element (2) umfasst.

6. Verfahren nach einem der vorangehenden Ansprüche, wobei das Verfahren den Schritt des Heißsiegelns des Abdeckstreifens (7) an das vordere Element (3) mittels eines inneren Siegels (14) umfasst, das zwischen einem oberen Rand (5) des vorderen Elements (3) und einem unteren vorderen Rand (16) des Abdeckstreifens (7) liegt.

7. Verfahren nach Anspruch 6, wobei das innere Siegel (14) im Wesentlichen parallel zu dem oberen Rand (5) und einem unteren vorderen Rand (16) ist.

8. Verfahren nach einem der vorangehenden Ansprüche, wobei die innere Thermoplastschicht (26) eine Zahl von Ritzen oder Lochungen (27) aufweist, um das Hindurchgelangen von Gas zuzulassen.

9. Verfahren nach einem der vorangehenden Ansprüche, wobei es sich bei der gasdurchlässigen Membran um ein Papier in chirurgischer Qualität handelt, es sich bei der inneren Thermoplastschicht um einen gelochten abziehbaren Polyolefinfilm handelt und es sich bei dem vorderen und dem hinteren Element um Polyethylenmaterial niedriger Dichte in medizinischer Qualität handelt.

## Revendications

1. Procédé de fabrication d'une poche plastique stérilisable comprenant les étapes consistant à :
former une bande de recouvrement détachable par pelage (7) par thermoscellage d'une membrane extérieure perméable aux gaz (25) sur une couche intérieure de film thermoplastique perméable aux gaz (26) ;
recouvrir la bande de recouvrement (7) par chevauchement au moyen d'un panneau thermoplastique avant (3) de la poche, un bord supérieur (5) du panneau avant (3) étant positionné de manière intermédiaire entre un bord supérieur (35) et un bord inférieur (16) de la bande de recouvrement (7) ;
thermosceller la bande de recouvrement (7) sur le panneau avant (3) ;
ledit procédé étant **caractérisé en ce qu'**il comporte par ailleurs les étapes consistant à :
poser un panneau thermoplastique arrière (2) sur le panneau avant (3) et la bande de recouvrement (7) ; et
thermosceller le panneau arrière (2) sur le panneau avant (3) et la bande de recouvrement (7) le long de joints d'étanchéité (10) des bords latéraux et d'un joint d'étanchéité (11) du bord supérieur.

2. Procédé selon la revendication 1, dans lequel le procédé comprend l'étape consistant à thermosceller (30, 31, 32) la membrane perméable aux gaz (25) sur la couche intérieure de film thermoplastique (26) autour d'une périphérie de la membrane (25) et de la couche intérieure de film thermoplastique (26).

3. Procédé selon la revendication 1 ou la revendication 2, dans lequel le procédé comprend l'étape consistant à thermosceller (14) la bande de recouvrement (7) sur le panneau avant (3) de manière intermédiaire entre les extrémités des parties recouvertes par chevauchement de manière espacée par rapport à un bord avant libre (16) de la bande de recouvrement (7).

4. Procédé selon la revendication 1, dans lequel le procédé comprend l'étape consistant à thermosceller la membrane perméable aux gaz (25) sur la couche intérieure thermoplastique (26), la couche intérieure thermoplastique faisant saillie vers l'extérieur depuis la membrane perméable aux gaz pour former un rabat de saisie (18).

5. Procédé selon l'une quelconque des revendications précédentes, dans lequel le procédé comprend l'étape consistant à thermosceller la membrane perméable aux gaz (25) sur la couche intérieure thermoplastique (26) le long de joints d'étanchéité latéraux (30) et d'un joint d'étanchéité inférieur (32) avant d'attacher la bande de recouvrement (7) sur le panneau arrière (2).

6. Procédé selon l'une quelconque des revendications précédentes, dans lequel le procédé comprend l'étape consistant à thermosceller la bande de recouvrement (7) sur le panneau avant (3) au moyen d'un joint d'étanchéité intérieur (14) qui est situé de manière intermédiaire entre un bord supérieur (5) du panneau avant (3) et un bord avant inférieur (16) de la bande de recouvrement (7).

7. Procédé selon la revendication 6, dans lequel le joint d'étanchéité intérieur (14) est sensiblement parallèle par rapport au bord supérieur (5) et au bord avant inférieur (16).

8. Procédé selon l'une quelconque des revendications précédentes, dans lequel la couche intérieure thermoplastique (26) a un certain nombre d'entailles ou de perforations (27) pour permettre le passage de gaz au travers de celle-ci.

9. Procédé selon l'une quelconque des revendications précédentes, dans lequel la membrane perméable aux gaz est un papier de qualité chirurgicale, la couche intérieure thermoplastique est un film polyoléfinique perforé détachable par pelage et les panneaux avant et arrière sont réalisés en un matériau de polyéthylène basse densité de qualité médicale.
